# EUROPÄISCHE PATENTSCHRIFT

(11) **EP 0 824 701 B1**
(45) Veröffentlichungstag und Bekanntmachung des Hinweises auf die Patenterteilung: **04.10.2000**
(21) Anmeldenummer: 96919548.6
(22) Anmeldetag: 07.05.1996
(51) Int. Cl.: G01N 33/74, G01N 33/574, G01N 33/531

(54) **VERFAHREN ZUM ORTSAUFGELÖSTEN IMMUNHISTOCHEMISCHEN NACHWEIS VON STEROIDREZEPTOREN**
METHOD FOR THE LOCALLY-RESOLVED IMMUNE-HISTOCHEMICAL DETECTION OF STEROID RECEPTORS
PROCEDE DE DETECTION IMMUNOHISTOCHIMIQUE, A RESOLUTION LOCALE, DE RECEPTEURS STEROIDIQUES

(30) Priorität: 12.05.1995 DE 19516954
(43) Veröffentlichungstag der Anmeldung: 25.02.1998
(73) Patentinhaber: JUNGBLUT, Peter, Prof.Dr.Dr., 34535 Neustadt (DE); Sierralta, Walter, 30625 Hannover (DE)
(72) Erfinder: JUNGBLUT, Peter, Prof.Dr.Dr., 34535 Neustadt (DE); Sierralta, Walter, 30625 Hannover (DE)
(74) Vertreter: Jungblut, Bernhard Jakob
(86) Internationale Anmeldenummer: DE9600867
(87) Internationale Veröffentlichungsnummer: WO9635956

(56) Entgegenhaltungen:
- EP-A- 0 129 669
- EP-A- 0 311 368
- WO-A-88/08537
- US-A- 5 292 638
- NATURE, Bd. 307, 23.Februar 1984, LONDON GB, Seiten 745-747, XP002013276 W. J. KING ET AL.: "Monoclonal antibodies localize oestrogen receptor in the nuclei of target cells." in der Anmeldung erwähnt

## Beschreibung

Die Erfindung betrifft ein Verfahren zum ortsaufgelösten immunhistochemischen Nachweis von Steroidrezeptoren in Zielzellen enthaltendem Gewebe, wobei eine Gewebeprobe entwässert und in einer üblichen Einbettmasse eingebettet wird, wobei von der eingebetteten Gewebeprobe sequentielle Gewebeschnitte angefertigt werden, wobei zumindest ein Teil der Gewebeschnitte mit Antikörpern inkubiert wird und wobei angelagerte Antikörper sichtbar gemacht werden. - Steroide wirken in sogenannten steroidabhängigen Zellen als Konformationskatalysatoren für die die Transkription regulierenden Steroidrezeptoren. Die die Transkription regulierende Wirkung wird dabei vom Steroid/Steroidrezeptor-Komplex als solchem ausgeübt. Es versteht sich, daß für diese Wirkung bindungsfähiger Steroidrezeptor in einer Zelle vorliegen muß, und daß damit das Steroid aus dem Blut und/oder der Gewebeflüssigkeit angereichert werden muß. Als Zielzellen sind entweder steroidabhängige Tumorzellen, in welchen die Proliferation der Tumorzellen von der Anwesenheit von Steroiden abhängt, oder solche gesunde Zellen, in welchen der Steroid/Steroidrezeptor-Komplex letztlich eine normale Transkription auslöst, bezeichnet. Solche gesunden Zellen bilden beispielsweise die primären und sekundären Geschlechtsorgane beim Menschen.

Ein Verfahren der eingangs genannten Art ist beispielsweise aus der Literaturstelle W.I. King, G.L. Greene; Nature, 1984, Band 307, S. 745 bis 747, bekannt. Dabei wird der immunhistochemische Nachweis von steroidbeladenem Steroidrezeptor und/oder unbeladenem Steroidrezeptor direkt mittels monoklonaler Antikörper (oder direkt mittels spezifischer, polyklonaler Antikörper) und folgender Analytik mittels Färbemethoden durchgeführt, wobei in einem Gewebeschnitt steroidrezeptorhaltige Gewebebereiche sichtbar werden. In Verbindung mit einer pathologischen Evaluierung der Gewebeschnitte, das heißt der Bestimmung jener Zellen, die Tumorzellen sind, läßt sich im Idealfall feststellen, ob die Tumorzellen steroidabhängig sind oder nicht. Mit dieser Feststellung kann eine Therapiestrategie zur Bekämpfung des Tumors aufgestellt werden. Bei steroidabhängigen Tumorzellen kann die Proliferation durch Blockierung des Steroidrezeptors, beispielsweise mittels Antiestrogene, gehemmt werden. Bei nicht steroidabhängigen Tumorzellen muß dagegen mit einer nicht hormonellen Therapie gearbeitet werden. Das insofern bekannte Verfahren ist jedoch mit Nachteilen behaftet. Einerseits werden Steroidrezeptoren, welche für die Antikörper unzugängliche Rezeptorepitope besetzen, nicht erfaßt. Andererseits werden solche (zugängliche) Steroidrezeptoren ungewollt erfaßt, die ihre Bindungsfähigkeit für das Steroid, beispielsweise mutationsbedingt, verloren haben. Beides kann zu beachtlichen Fehlinterpretationen des gefundenen Ergebnisses führen. Im übrigen sind die erforderlichen monoklonalen (oder spezifischen, polyklonalen) Antikörper sehr teuer.

Der Erfindung liegt das technische Problem zugrunde, ein Verfahren zum ortsaufgelösten immunhistochemischen Nachweis von Steroidrezeptoren in Zielzellen enthaltendem Gewebe anzugeben, welches mit besonders hoher Zuverlässigkeit alle Steroidrezeptoren erfaßt, die (noch) bindungsfähig bezüglich des Steroides sind, und welches gleichzeitig vergleichsweise preiswert durchzuführen ist.

Zur Lösung dieses Problems lehrt die Erfindung ein Verfahren zum ortsaufgelösten immunhistochemischen Nachweis von Steroidrezeptoren in Zielzellen enthaltendem Gewebe mit folgenden Verfahrensstufen: Eine Gewebeprobe mit Zielzellen wird mit einer wäßrigen Formaldehyd-Lösung deren pH-Wert weniger als 7,0 beträgt, behandelt, wobei ein nicht kovalent gebundener Ligand des Steroidrezeptors vom Steroidrezeptor freigesetzt und in molekularer Nähe an einer sterisch offenen Bindungsstelle kovalent gebunden wird. Danach wird die Gewebeprobe entwässert und in einer üblichen Einbettmasse eingebettet. Von der eingebetteten Gewebeprobe werden sequentielle Gewebeschnitte angefertigt. Danach werden zumindest ein Teil der Gewebeschnitte rehydriert und mit ligandspezifischen Antikörpern inkubiert, wobei die ligandspezifischen Antikörper an an sterisch offenen Bindungsstellen gebundene Liganden angelagert werden. Die angelagerten ligandspezifischen Antikörper werden mittels eines zweiten Antikörper/Enzym-Konjugats und durch dessen Umsatz mit Substrat zu einem unlöslichen Produkt sichtbar gemacht. - Der pH-Wert sollte jedenfalls unterhalb von 7,0 liegen. Als Liganden sind solche Substanzen bezeichnet, die an die Steroidrezeptoren binden können. Liganden können Steroidrezeptors auslösen, sein. Steroide sind beispielsweise Estrogene und Androgene. Eines der Estrogene ist Estradiol. Antiestrogene sind beispielsweise 4-Hydroxitamoxifen und Desmethylnafoxidin. Eines der Androgene ist Dihydrotesteron. Ein Antiandrogen ist beispielsweise Cyproteronacetat. Sterisch offene Bindungsstellen des Liganden sind solche Bindungsstellen, an denen der kovalent gebundene Ligand zugänglich für die ligandspezifischen Antikörpern ist. Als zweite Antikörper sind solche Antikörper bezeichnet, die selbst wiederum als Antikörper für die ligandspezifischen Antikörpern wirken. Die zur Durchführung des erfindungsgemäßen Verfahrens erforderlichen Antikörper sind käuflich oder mit wenig Aufwand herzustellen und insgesamt vergleichsweise sehr preiswert.

Die Erfindung beruht auf der Erkenntnis, daß aktive, also bindungsfähige Steroidrezeptoren dadurch lokalisiert werden können, daß von aktiven Steroidrezeptoren, und zwar auch von für einen direkten Antikörperzugang unzugänglichen Steroidrezeptoren, das gebundene'Steroidmolekül gelöst und an eine in molekularer Nähe liegende, zugängliche Stelle gleichsam umgelagert werden können. Diese Umlagerung erfolgt im Sauren und mit Formaldehyd, wobei mittels des Formaldehyds eine kovalente Bindung des Steroids beispielsweise an Amino- oder Iminogruppen in Seitenketten von ein Protein bildenden Aminosäuren über eine Mannich-Reaktion (vor allem an Epsilon-Aminolysyl-Seitenketten) bewirkt wird. Dabei erfolgt die kovalente Bindung in molekularer Nähe zum Steroidrezeptor, aus welchem das Steroid im Sauren freigesetzt wurde. Es versteht sich, daß die Bedingungen, insbesondere pH-Wert und Temperatur in der Verfahrensstufe a), so gewählt und abgestimmt werden, daß ein sogenanntes Verwaschen durch lange Diffusionswege des Liganden zwischen dem zugeordneten Steroidrezeptor und der (späteren) kovalenten Bindungsstelle des vom Steroidrezeptor freigesetzten Liganden nicht in störendem Maße stattfindet. Ein Verwaschen um nicht mehr als 10 Mikrometer ist für eine brauchbare Auswertung gerade noch akzeptabel, weniger als 5 Mikrometer sind anzustreben. Daher umfaßt der Begriff "molekulare Nähe" im Sinne der Erfindung auch noch eine Entfernung von ca. 5 Mikrometer, wenn auch zweckmäßigerweise der pH-Wert und die Temperatur so abgestimmt sind, daß die Entfernung im Sub-Mikrometerbereich, vorzugsweise unter einigen 10 Nanometern, liegt.

Im Ergebnis läßt sich in Verbindung mit einer pathologischen Evaluierung des gleichen oder anderer sequentieller Gewebeschnitte feststellen, ob die im Zuge der Evaluierung identifizierten Tumorzellen das Steroid wie gesunde Zellen angereichert haben oder nicht. Insbesondere bei Mischsituationen Tumorgewebe/gesundes Gewebe sind aufgrund der mikroskopischen Ortsauflösung somit eindeutige Ergebnisse erhältlich, wobei zudem sichergestellt ist, daß nur aktive, d.h. bindungsfähige Steroidrezeptoren erfaßt werden, und zwar unabhängig von der Lage der Rezeptorepitope. Bei einer Anreicherung des Steroids im Tumorgewebe liegt ein steroidabhängiger Tumor vor. Umgekehrt kann aber auch Tumorgewebe identifiziert werden, welches einen nicht steroidabhängigen Tumor bildet. Dies gilt insbesondere, wenn die Gewebeprobe einem Gewebe entstammt, dessen gesunde Zellen natürlicherweise beachtliche Steroidmengen binden können. Das erfindungsgemäße Verfahren ist im übrigen sehr empfindlich.

So hat sich beispielsweise gezeigt, daß die niedrigen natürlichen Estradiolkonzentrationen im Plasma klimakterischer Frauen sowie von Männern in der Regel für den Nachweis einer Estradiolanreicherung in estradiolabhängigen Tumorzellen gegenüber den gesunden Zellen bereits ausreichen. Vorsorglich kann jedoch auch kurz vor oder während der Entnahme der Gewebeprobe Ligand, beispielsweise 10 Mikrogramm Estradiol oder eines der Antiestrogene, intravenös injiziert werden.

In vorteilhafter Weiterbildung der Erfindung wird die Behandlung der Gewebeprobe mit einer Lösung von z.B. 5 Vol.-%, in der Regel 4 Vol.-%, Formaldehyd und 10 Vol.-% Essigsäure bzw. Eisessig in Wasser durchgeführt. Als Einbettmasse kann Paraffin verwendet werden. Als ligandspezifische Antikörper können solch von der Ziege verwendet werden, wobei die Antikörper in einem üblichen Lösungsmittel gelöst und stabilisiert sind. Als zweites Antikörper/Enzym-Konjugat ist beispielsweise ein mit Meerrettichperoxidase konjugiertes Antiziegen-Immunglobulin vom Kaninchen geeignet. Als Substrat kann dann Diaminobenzidin/H₂O₂ verwendet werden. Gegenstand der Erfindung ist auch die Verwendung von für Liganden des Steroidrezeptors spezifischen Antikörpern zur Herstellung eines Reagenz für ein Verfahren nach einem der Ansprüche 1 oder 2.

Für andere Zwecke als den indirekten Nachweis von Steroidrezeptoren ist aber auch Gegenstand der Erfindung ein Verfahren gemäß Patentanspruch 4, mit welchem Steroide direkt immunhistochemisch und mit guter Auflösung unabhängig von dem Vorhandensein von Steroidrezeptoren nachweisbar sind. Zwar ist es aus der Praxis bekannt, Proteine nach einer Fixierung mit proteinspezifischen Antikörpern immunhistochemisch nachzuweisen, jedoch ist es bislang nicht erkannt worden, daß auch Steroide so fixierbar sind, daß sie beispielsweise unter Inkubationsbedingungen nicht verwaschen bzw. komplett auswaschen. Daher ist zum ortsaufgelösten Nachweis von Steroiden bislang auf radiografische Methoden mit speziellen, bei immunhistochemischen Verfahren unbrauchbaren Fixierungsmethoden zurückgegriffen worden, wobei die gewonnenen Ergebnisse bereits deshalb fragwürdig sind, weil beispielsweise mit radioaktivem Jod markierte Steroide sich anders verhalten als die natürlichen Steroide. Zudem stört die Radioaktivität als solche aus Strahlenbelastungsgründen. Im Ergebnis besteht die Grundkonzeption der Erfindung nicht nur darin, überhaupt erstmals steroidspezifische (bzw. antisteroidspezifische) Antikörper für ein immunhistochemisches Verfahren zu nutzen, sondern in Verbindung damit erstmals eine für die Fixierung von Steroiden (bzw. Antisteroiden) in einem immunhistochemischen Verfahren geeignete Fixierungsverfahrensstufe anzugeben. Die Erfindung fünktioniert dabei grundsätzlich mit allen solchen Liganden, die ein phenolisches Hydroxyl aufweisen und nach Mannich reaktiv sind. Beispiele hierfür sind: 17-alpha-Estradiol, Östriol, Östron, 6-alpha-Hydroxyöstron und 7-alpha-Hydroxyöstron. Im übrigen treffen auch die Erläuterungen zu den Patentansprüchen 1 bis 3 entsprechend zu, sofern sie nicht Steroidrezeptoren selbst betreffen. Gegenstand der Erfindung ist schließlich auch die Verwendung gemäß Patentanspruch 5.

Das Verfahren nach Anspruch 4 läßt sich für verschiedene Zwecke nutzen, wie beispielsweise in den Patentansprüchen 6 und 7 angegeben. So können in Muskelgewebe das Muskelwachstum stimulierende Substanzen auf Steroidbasis nachgewiesen werden. Ebenso kann eine Bestimmung des Muskelwachstums durchgeführt werden, wobei Wachstumsraten durch Auszählen von sichtbar gemachten Satellitenzellen in einer Muskelgewebeprobe ermittelt werden. In beiden Fällen werden folgende Zusammenhänge genutzt. Zelluläre Einheit des quergestreiften Muskels ist die Faser, ein Syncytium bestehend aus zentralem Aktomyosinbündel und aufliegenden, nicht mehr teilungsfähigen Kernen. Wachstumsimpulse erfolgen über noch teilungsfähige, sogenannte Satellitenzellen, die der Fasermembran außen anliegen. Da ihr Kern nur von einem schmalen Cytoplasmasaum umgeben ist, bedarf es elektronenmikroskopischer Auflösung, um zwischen Satellitenzellen auf der Fasermembran und Syncytiumkernen an der Innenseite zu unterscheiden. Satellitenzellen enthalten Rezeptoren für anabole Steroide und besitzen deshalb - in allen Zielzellen - ein beträchtliches Anreicherungsvermögen für Estradiol sowie für andere (natürliche wie synthetische) rezeptoraffine Liganden. Erfindungsgemäß können folglich nicht-natürliche Liganden, beispielsweise aufgrund der Gabe von solchen Liganden in der Nutztiermast (Fleischproduktion) oder im Leistungsport, dadurch nachgewiesen werden, daß die Satellitenzellen in dem Gewebeschnitt sichtbar werden, wenn mit für die nicht-natürlichen Liganden spezifischen Antikörpern gearbeitet wird. Nicht-natürlich meint hierbei solche Liganden, die natürlicherweise nicht in dem untersuchten Gewebe vorkommen. Aber auch eine Gabe natürlicher Liganden ist nachweisbar, wenn die mittels entsprechender Antikörper sichtbar gemachten Satellitenzellen ausgezählt werden und ihre Zahl mit der Zahl von Satellitenzellen in dem betreffenden Gewebe unter normalen Wachstumsbedingungen verglichen werden. Im Ergebnis sind diese erfindunggemäßen Verwendungen sehr einfache, hochempfindliche und wirksame Verfahren, um Mißbrauch von Steroiden in der Nutztiermast (Fleischproduktion) oder beim Leistungssport nachzuweisen. Dabei ist es unerheblich, ob natürliche Substanzen, wie Zeranol oder Genistein, oder synthetische Substanzen, wie Diethylstilbestrol oder 17-alpha-Ethinylestradiol, nachzuweisen sind, solange es sich um rezeptoraffine Substanzen handelt. Dazu ist es lediglich notwendig eine Gewebeprobe zu erhalten, wobei eine nennenswerte Beeinträchtigung des lebenden Gewebespenders und/oder des zu untersuchenden Gewebes nicht eintritt, wenn beispielsweise mittels der Nadelbiopsie gearbeitet wird. Demgegenüber ist es zwar bekannt zum Nachweis einer erhöhten Zahl von Satellitenzellen in der Nutztiermast deren Anreicherungsvermögen für Bromdeoxyuridin zu nutzen (E. Schultz et al., J. Applied Physiol., 76, 1994, 266-270), dabei ist allerdings die subsequente Schlachtkörperverwertung eingeschränkt bzw. ausgeschlossen, was insbesondere bei negativem Befund in erheblichem Maße stört.

In jeder Ausbildung der Erfindung wird vorteilhafterweise mit ligandspezifischen Antikörpern gearbeitet, die dadurch erhältlich sind, daß ein Trägerprotein, vorzugsweise Rinderserumalbumin, mit dem Liganden zu einem Antigen gekoppelt wird, das Antigen von störenden Substanzen aus der Koppelung abgetrennt wird, mit dem abgetrennten Antigen eine Immunisierung eines zur Bildung von ligandspezifischen Antikörpern fähigen Organismus durchgeführt wird und dem immunisierten Organismus Blut abgenommen wird, woraus ein die ligandspezifischen Antikörper enthaltendes Serum gebildet wird. Danach können erforderlichenfalls die ligandspezifischen Antikörper aus dem Serum in üblicher Weise aufgereinigt, in einem üblichen Lösungsmittel gelöst und stabilisiert werden.

Im folgenden wird die Erfindung anhand von Ausführungsbeispielen näher erläutert. In allen Ausführungsbeispielen wurde die Gewebeprobe sofort nach der Entnahme in 10 Vol.-% Eisessig und 4 Vol.-% Formaldehyd (Rest: Wasser) bei Zimmertemperatur für drei bis fünf Stunden fixiert. Danach wurde die Gewebeprobe entwässert, in Paraffin eingebettet und geschnitten. Sequentielle Gewebeschnitte wurden entparaffiniert und gefärbt (zwecks pathologischer Evaluierung) bzw. rehydriert, mit H₂O₂ vorinkubiert und dann mit estradiolbindendem Antikörper von der Ziege für ca. 2 Stunden bei Raumtemperatur inkubiert. Anschließend wurde mit Meerettichperoxydase konjugiertes Antiziegen-Immunglobulin vom Kaninchen zugesetzt. Als Substrat diente Diaminobenzidin. Kontrollinkubationen zur Kontrolle für die Immunreaktion erfolgten mit Nicht-Immun/Ziegen IgG. Wie üblich, wurden die Gewebeschnitte mit Hämatoxilin gegengefärbt.

### Ausführungsbeispiel 1

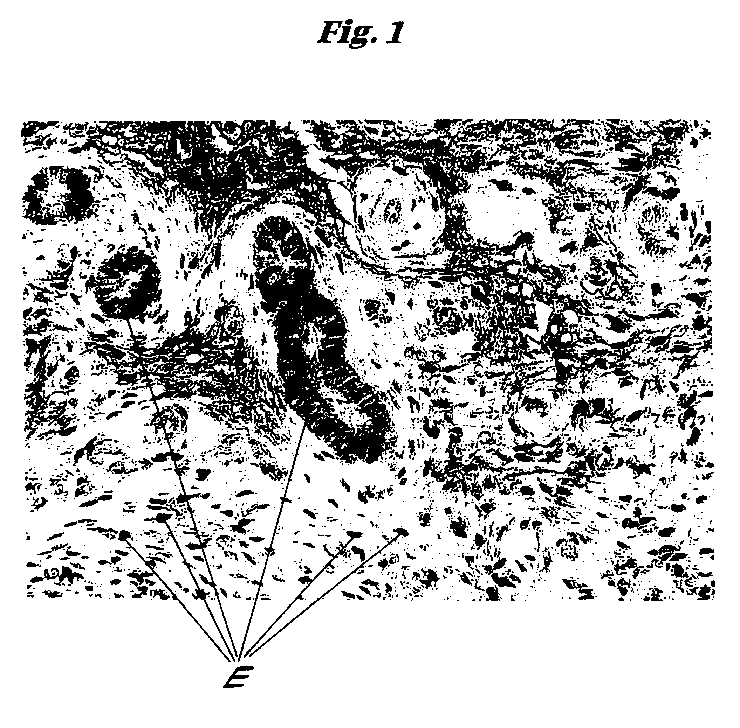

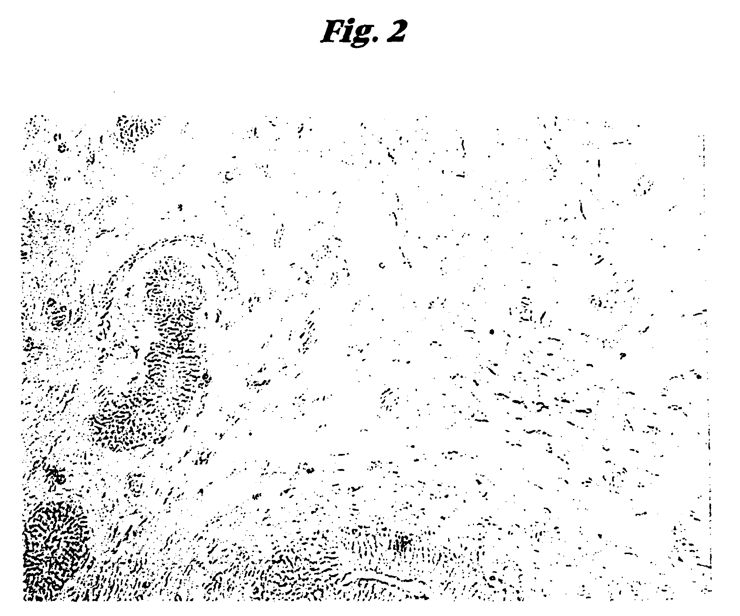

Untersucht wurde ein Schweineuterus, wobei die Entnahme der Gewebeprobe 5 Minuten nach intrauteriner Gabe von 25 ml pro Uterushorn einer Lösung von 0,27 Mikrogramm Estradiol je ml erfolgte. Die Fig. 1 zeigt einen Gewebeschnitt nach Durchführung des erfindungsgemäßen Verfahrens mit einer Immunreaktion für Estradiol und in der Fig. 2 ist ein Gewebeschnitt dargestellt, an welchem die Kontrolle für die Immunreaktion durchgeführt wurde. Die Vergrößerung beträgt 400-fach. In der Fig. 1 erkennt man deutlich dunkelgefärbte Bereiche und Strukturen, während solche Bereiche in der Fig. 2 fehlen. Beispiele der dunklen Bereiche und Strukturen in der Fig. 1 sind mit dem Bezugszeichen E versehen. Die dunkelgefärbten Bereiche und Strukturen E sind in farblichen Darstellungen der Gewebeschnitte bräunlich gefärbt. Die dunkelgefärbten Bereiche und Strukturen E in der Fig. 1 gehen ausweislich der Kontrolle, Fig. 2, auf die estradiolspezifische Immunreaktion des erfindungsgemäßen Verfahrens zurück. In der Fig. 1 ist aber auch deutlich zu erkennen, daß die Umlagerung des vom Steroidrezeptor freigesetzten Steroides in molekularer Nähe des Steroidrezeptors stattfindet, da die dunkelgefärbten Bereiche und Strukturen E scharf abgegrenzt und nicht etwa "verwaschen" sind.

### Ausführungsbeispiel 2

Untersucht wurde eine Gewebeprobe aus der Brust einer 71-jährigen Frau mit Brusttumor. Eine quantitativ-analytische Untersuchung ergab 22 pg Estradiol/ml Plasma und 253 pg Estradiol/g Tumor. Je ml Gewebeextrakt wurden 2480 fmol Estradiolrezeptor und 4120 fmol Progesteronrezeptor gefunden.

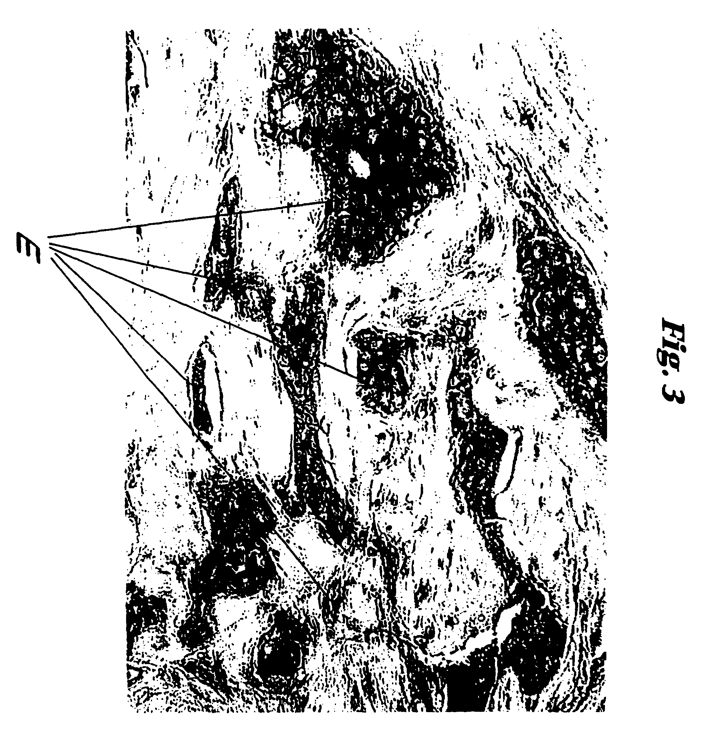

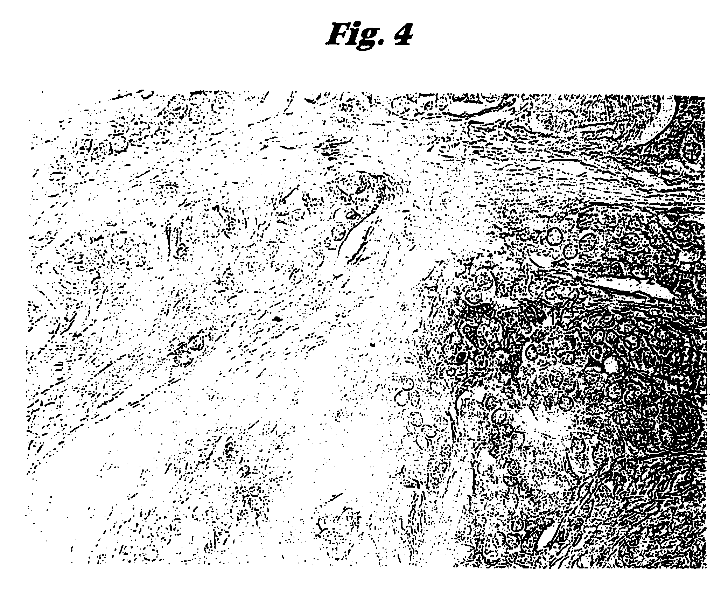

Diese analytischen Werte deuten darauf hin, daß der Tumor zumindest zum Teil aus steroidabhängigem Tumorgewebe besteht. Die Fig. 3 und 4 zeigen Gewebeschnitte nach Durchführung des erfindungsgemäßen Verfahrens mit Immunreaktion für Estradiol (Fig. 3) sowie aus der Immunkontrolle (Fig. 4) mit 400-facher Endvergrößerung. In der Fig. 3 erkennt man dunkle Bereiche und Strukturen E (tatsächlich von bräunlicher Färbung), während solche Bereiche und/oder Strukturen in der Fig. 4 nicht erkennbar sind. Dies belegt, daß die pathologisch identifizierten Tumorzellen Estradiol anreichern. Folglich sind diese Tumorzellen estradiolabhängig und eine Therapie mit Antiestrogenen kann möglicherweise zur Inhibierung des Tumorwachstumes führen.

### Ausführungsbeispiel 3

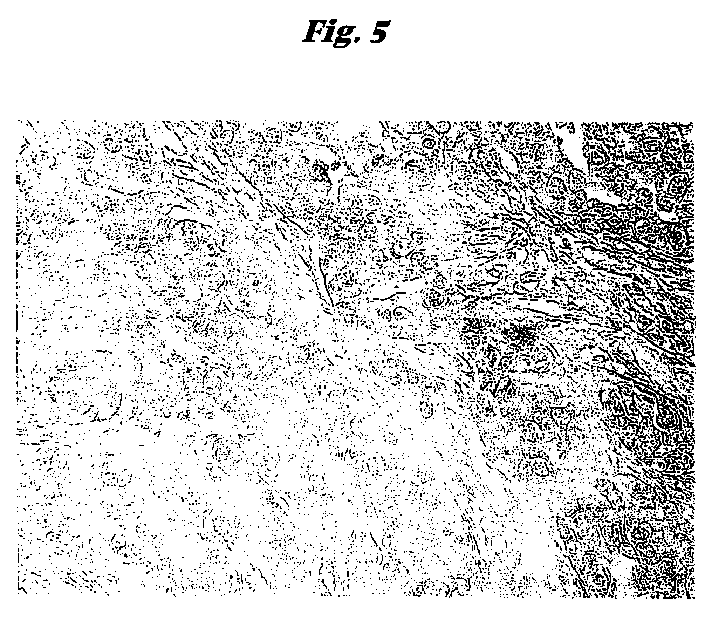

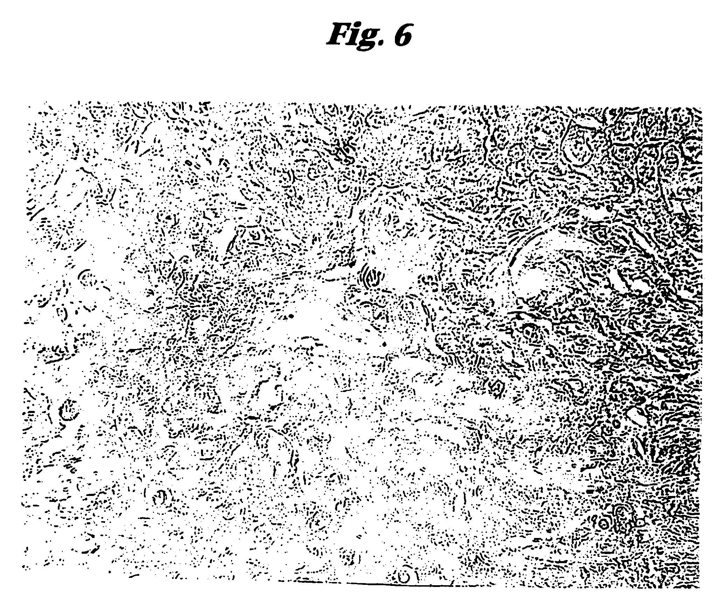

Untersucht wurde eine Gewebeprobe aus der Brust einer 66-jährigen Frau mit Brusttumor. Eine quantitativ-analytische Untersuchung ergab 12 pg Estradiol/ml Plasma und 0 pg Estradiol/g Tumor. Je ml Gewebeextrakt wurden 0 fmol Estradiolrezeptor und 0 fmol Progesteronrezeptor gefunden. Diese analytischen Werte belegen, daß der Tumor insgesamt aus nicht steroidabhängigem Tumorgewebe besteht. Die Fig. 5 und 6 zeigen Gewebeschnitte nach Durchführung des erfindungsgemäßen Verfahrens (Fig. 5) sowie aus der Immunkontrolle (Fig. 6) mit 400-facher Endvergrößerung. Deutlich zu erkennen ist, daß weder bei der Immunkontrolle noch bei der Immunreaktion für Estradiol ein positives Ergebnis erhalten wurde. Dies belegt, daß die pathologisch identifizierten Tumorzellen Estradiol nicht anreichern. Folglich sind diese Tumorzellen nicht estradiolabhängig und eine Therapie mit Antiestrogenen wäre vermutlich sinnlos.

### Ausführungsbeispiel 4

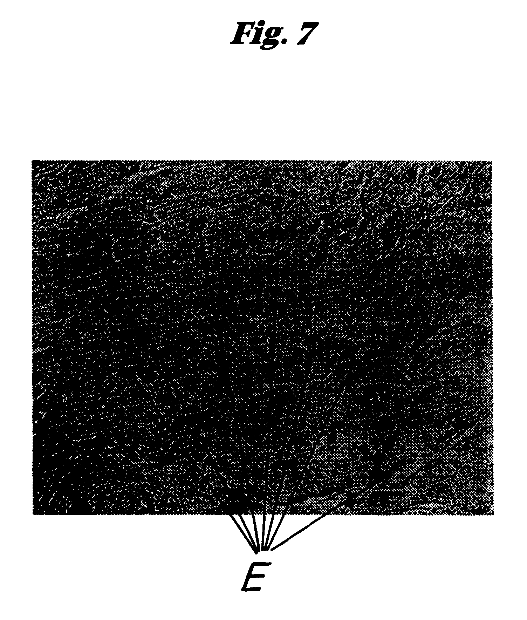

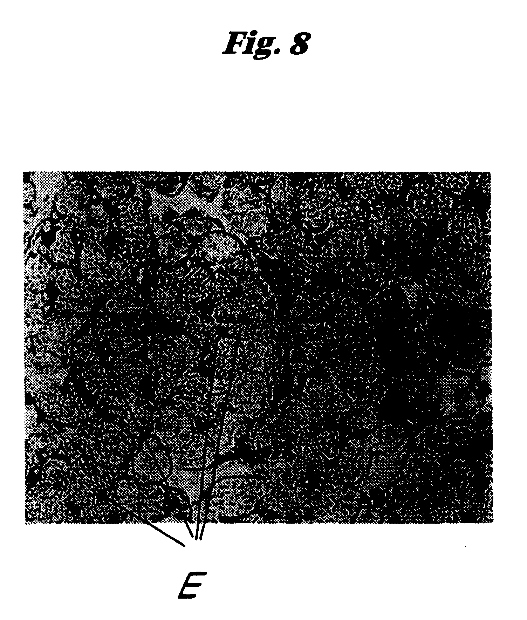

Aus dem M. semitendinosus eines 10 Tage alten männlichen Göttinger Miniaturschweines wurde eine Gewebeprobe genommen. Eine quantitativ-analytische Untersuchung des Blutplasmas ergab eine Estradiolkonzentration von weniger als 0,1 pg/ml. Nach Durchführung des erfindungsgemäßen Verfahrens mit für Estradiol spezifischen Antikörpern wurden die Gewebeschnitte gemäß der Figuren 7 und 8 erhalten. Figur 7 ist ein Schnitt parallel zu den Aktomyosinbündel und Figur 8 ein Schnitt orthogonal dazu. In beiden Fällen erkennt man sehr gut mit hoher Ortsauflösung die dunkel gefärbten Satellitenzellen E, wodurch Estradiol in den Satellitenzellen trotz der extrem geringen Plasmakonzentration nachgewiesen ist. Durch Auszählen der Satellitenzellen kann auf (ggf. unnatürliches) Muskelwachstum geprüft werden. Durch Verwendung anderer, entsprechend spezifischer Antikörper kann auf sonstige anabole Gaben untersucht werden.

Folgend wird schließlich beispielhaft erläutert, wie spezifische Antikörper im einzelnen erhältlich sind. 65 mg Rinderserumalbumin wurden in 0,6 ml Wasser und 0,6 ml Eisessig gelöst. Hierzu wurde 1 ml einer Lösung von 5 mg Estradiol in 1 ml Ethanol zugegeben, wobei das Estradiol als Spur tritiummarkiert war. Die Kopplung des Rinderserumalbumins mit dem Estradiol zum Antigen erfolgte dann durch Zugabe von 0,1 ml einer 37 %igen Formaldehydlösung. Durch Gelfiltration mit einer Sephadex G 25 Säule wurde nicht gekoppeltes Estradiol abgetrennt. Dabei konnte durch Untersuchung der Filtrationsfraktionen mit 280 nm Photometrie sowie mit Szintillationzählung sichergestellt werden, daß nur Antigen (sowie freies Rinderserumalbumin) der weiteren Verarbeitung zugeführt wurde. Mit dem insofern aufgereinigten Antigen wurden Kaninchen durch mehrfache multiple site Injektionen i.c. (intracutan) immunisiert, wobei mit einem üblichen Adjuvanz zur Stimulierung der Immunreaktion gearbeitet wurde. Nach Bestimmung eines ausreichenden Titers wurde den Kaninchen Blut abgenommen und daraus Serum gewonnen. Dieses Serum enthielt die für Estadiol spezifischen Antikörper in allen Anforderungen genügender Menge.

## Patentansprüche

1. Verfahren zum ortsaufgelösten immunhistochemischen Nachweis von Steroidrezeptoren in Zielzellen enthaltendem Gewebe mit folgenden Verfahrensstufen:
a) Eine Gewebeprobe mit Zielzellen wird mit einer wäßrigen Formaldehyd-Lösung deren pH-Wert weniger als 7,0 beträgt, behandelt, wobei ein nicht kovalent gebundener Ligand des Steroidrezeptors vom Steroidrezeptor freigesetzt und in molekularer Nähe an einer sterisch offenen Bindungsstelle kovalent gebunden wird.
b) Danach wird die Gewebeprobe entwässert und in einer üblichen Einbettmasse eingebettet.
c) Von der eingebetteten Gewebeprobe werden sequentielle Gewebeschnitte angefertigt.
d) Danach werden zumindest ein Teil der Gewebeschnitte rehydriert und mit ligandspezifischen Antikörpern inkubiert, wobei die ligandspezifischen Antikörper an an sterisch offenen Bindungsstellen gebundenen Ligand angelagert werden.
e) Die angelagerten ligandspezifischen Antikörper werden mittels eines zweiten Antikörper/Enzym-Konjugats und durch dessen Umsatz mit Substrat zu einem unlöslichen Produkt sichtbar gemacht.

2. Verfahren nach Anspruch 1, wobei die Behandlung der Gewebeprobe mit einer Lösung von 4 Vol.-% Formaldehyd und 10 Vol.-% Essigsäure in Wasser erfolgt, wobei als Einbettmasse Paraffin verwendet wird, wobei als ligandspezifische Antikörper Estradiol bindende Antikörper von der Ziege verwendet werden und wobei als zweiter Antikörper/Enzym-Konjugat ein mit Meerrettichperoxidase konjugiertes Antiziegen-Immunglobulin vom Kaninchen und als Substrat Diaminobenzidin/H₂O₂ verwendet werden.

3. Verwendung von für Liganden von Steroidrezeptoren spezifischen Antikörpern in einem Verfahren nach einem der Ansprüche 1 oder 2.

4. Verfahren zum ortsaufgelösten immunhistochemischen Nachweis von Steroiden in Zellen enthaltendem Gewebe mit folgenden Verfahrensstufen:
a) Eine Gewebeprobe wird mit einer wäßrigen Formaldehyd-Lösung deren pH-Wert weniger als 7,0 beträgt, behandelt, wobei nicht kovalent gebundene Steroidmoleküle freigesetzt und in molekularer Nähe an sterisch offenen Bindungsstellen kovalent gebunden werden.
b) Danach wird die Gewebeprobe entwässert und in einer üblichen Einbettmasse eingebettet.
c) Von der eingebetteten Gewebeprobe werden sequentielle Gewebeschnitte angefertigt.
d) Danach werden zumindest ein Teil der Gewebeschnitte rehydriert und mit steroidspezifischen Antikörpern inkubiert, wobei die steroidspezifischen Antikörper an an sterisch offenen Bindungsstellen gebundenen Steroidmolekülen angelagert werden.
e) Die angelagerten steroidspezifischen Antikörper werden mittels eines zweiten Antikörper/Enzym-Konjugats und durch dessen Umsatz mit Substrat zu einem unlöslichen Produkt sichtbar gemacht.

5. Verwendung von für Steroide spezifischen Antikörpern in einem Verfahren nach Anspruch 4.

6. Verwendung des Verfahrens nach Anspruch 4 zum Nachweis von das Muskelwachstum stimulierenden Liganden in Muskelgewebe.

7. Verwendung des Verfahrens nach Anspruch 4 zur Bestimmung des Muskelwachstums, wobei Wachstumsraten durch Auszählen von sichtbar gemachten Satellitenzellen in einer Muskelgewebeprobe ermittelt werden.

8. Verfahren nach einem der Ansprüche 1, 2 oder 4, wobei die ligandspezifischen Antikörper dadurch erhältlich sind, daß
a) ein Trägerprotein, vorzugsweise Rinderserumalbumin, mit dem Liganden zu einem Antigen gekoppelt wird,
b) das Antigen von störenden Substanzen aus der Koppelung abgetrennt wird
c) mit dem abgetrennten Antigen eine Immunisierung eines zur Bildung von ligandspezifischen Antikörpern fähigen Organismus durchgeführt wird und
d) dem immunisierten Organismus Blut abgenommen wird, woraus ein die ligandspezifischen Antikörper enthaltendes Serum gebildet wird.

9. Verfahren nach Anspruch 8, wobei die ligandspezifischen Antikörper aus dem Serum aufgereinigt, in einem üblichen Lösungsmittel gelöst und stabilisiert werden.

## Claims

1. A method for the position resolved immunohisto. chemical detection of steroid receptors in tissue containing target cells, comprising the following steps:
a) A tissue sample containing destination cells is treated with an aqueous formaldehyde solution with an index of pH of less than 7.0, whereby a not covalent-bound ligand of the steroid receptor is released from the steroid receptor and is covalent-bound in molecular proximity to a sterically open binding position.
b) Then the tissue sample is dewatered and embedded in an usual embedding mass.
c) From the embedded tissue sample sequential tissue cuts are produced.
d) Then at least part of the tissue cuts are rehydrated and incubated with ligand-specific antibodies, the ligand-specific antibodies being attached to a ligand bound to sterically open binding sites.
e) The attached ligand-specific antibodies are made visible by means of a second antibody-enzyme conjugate and by the conversion thereof with a substrate to an insoluble product.

2. A method according to claim 1, wherein the treatment of the tissue sample is performed in a solution of 4 % by vol. formaldehyde and 10 % by vol. acetic acid in water, wherein as embedding mass paraffin is used, wherein as ligand-specific antibodies goat antibodies binding estradiol are used and wherein as a second antibody-enzyme conjugate an anti-goat immunoglobulin from rabbit conjugated with horseradish peroxidase and as a substrate diaminobenzidin/H₂O₂ are used.

3. The utilization of antibodies specific for ligands of steroid receptors in a method according to one of claims 1 or 2.

4. A method for the position resolved immunohistochemical detection of steroids in tissue containing cells, comprising the following steps:
a) A tissue sample is treated with an aqueous formaldehyde solution with an index of pH of less than 7.0, whereby not covalent-bound steroid molecules are released and are covalent-bound in molecular proximity to sterically open binding positions.
b) Then the tissue sample is dewatered and embedded in an usual embedding mass.
c) From the embedded tissue sample sequential tissue cuts are produced.
d) Then at least part of the tissue cuts are rehydrated and incubated with steroid-specific antibodies, the steroid-specific antibodies being attached to steroid molecules bound to sterically open binding sites.
e) The attached steroid-specific antibodies are made visible by means of a second antibody-enzyme conjugate and by the reaction thereof with a substrate to an insoluble product.

5. The utilization of antibodies specific for steroids in a method according to claim 4.

6. The utilization of the method according to claim 4 for the detection of ligands stimulating muscle growth in muscle tissue.

7. The utilization of the method according to claim 4 for the determination of muscle growth, the growth rates being determined by counting satellite cells made visible in a muscle tissue sample.

8. A method according to one of claims 1, 2 or 4, wherein the ligand-specific antibodies are obtainable by that
a) a carrier protein, preferably bovine serum albumin, is coupled with the ligand to an antigen,
b) the antigen is separated from disturbing substances from the coupling,
c) with the separated antigen an immunization of an organism capable to form ligand-specific antibodies is performed, and
d) blood is taken from the immunized organism, wherefrom a serum containing the ligand-specific antibodies is produced.

9. A method according to claim 8, wherein the ligand-specific antibodies from the serum are cleaned, diluted in a usual solvent, and stabilized.

## Revendications

1. Procédé pour la détection immunohistochimique par localisation de récepteurs de stéroïdes dans un tissu contenant des cellules de destination,
comprenant les étapes suivantes:
a) Un fragment de tissu contenant des cellules de destination est traité avec une solution aqueuse de formaldehyde dont la valeur pH est inférieure à 7,0, un ligand non liée de façon covalente du récepteur de stéroïdes étant dégagé du récepteur de stéroïdes et lié de façon covalente à proximité moléculaire d'une position de liaison stériquement ouverte.
b) Puis le fragment de tissu est déshydraté et enrobé en une masse d'inclusion conventionnelle.
c) A partir de la masse d'inclusion sont exécutées des coupes séquentielles du tissu.
d) Puis au moins une partie des coupes de tissu est réhydratée et incubée avec des anticorps spécifiques pour les ligands, les anticorps spécifiques pour les ligands étant fixés à un ligand lié à des positions de liaison stériquement ouvertes.
e) Les anticorps fixés spécifiques pour les ligands sont visualisés au moyen d'un deuxième conjugat anticorps/ enzyme et par la conversion de celui-ci avec un substrat à un produit insoluble.

2. Procédé selon la revendication 1, dans lequel le traitement du fragment de tissu se fait avec une solution de 4 % vol. de formaldehyde et 10 % vol. d'acide acétique, paraffine est utilisé comme masse d'inclusion, des anticorps de chèvre liant l'estradiole sont utilisés comme anticorps spécifiques pour les ligands, et un immunoglobuline anti-chèvre du lapin conjugué avec peroxidase de raifort est utilisé comme deuxième conjugat anticorps/enzyme, et diaminobenzidine/H3O2 comme substrat.

3. Utilisation d'anticorps spécifiques pour les ligands de récepteurs de stéroïdes dans un procédé selon une des revendications 1 ou 2.

4. Procédé pour la détection immunohistochimique par localisation de stéroïdes dans un tissu contenant des cellules, comprenant les étapes suivantes:
a) Un fragment de tissu est traité avec une solution aqueuse de formaldehyde dont la valeur pH est inférieure à 7,0, des molécules non liées de façon covalente étant dégagées et liées de façon covalente à proximité moléculaire de positions de liaison stériquement ouvertes.
b) Puis le fragment de tissu est déshydraté et enrobé en une masse d'inclusion conventionnelle.
c) A partir de la masse d'inclusion sont exécutées des coupes séquentielles du tissu.
d) Puis au moins une partie des coupes de tissu est réhydratée et incubée avec des anticorps spécifiques pour les stéroïdes, les anticorps spécifiques pour les stéroïdes étant fixés à des molécules stéroïdiques liées à des positions de liaison stériquement ouvertes.
e) Les anticorps fixés spécifiques pour les stéroïdes sont visualisés au moyen d'un deuxième conjugat anticorps/ enzyme et par la conversion de celui-ci avec un substrat à un produit insoluble.

5. Utilisation d'anticorps spécifiques pour les stéroïdes dans un procédé selon la revendication 4.

6. Utilisation du procédé selon la revendication 4 pour la détection de ligands dans le tissu musculaire stimulant l'accroissement des muscles.

7. Utilisation du procédé selon la revendication 4 pour la détermination de l'accroissement des muscles, les taux d'accroissement se faisant par comptage des cellules satellites visualisées dans un fragment de tissu musculaire.

8. Procédé selon une des revendications 1, 2 ou 4, dans lequel les anticorps spécifiques pour les ligands sont obtenus par ce que:
a) une protéine porteuse, de préférence une albumine de sérum bovin, est couplée avec le ligand pour former un antigène,
b) l'antigène est dégagé de substances gênantes de l'accouplement,
c) avec l'antigène dégagé se fait une immunisation d'un organisme capable de former des anticorps spécifiques pour les ligands,
d) du sang est pris de l'organisme immunisé, donc un sérum contenant les anticorps spécifiques pour les ligands étant produit.

9. Procédé selon la revendication 8, dans lequel les anticorps spécifiques pour les ligands du sérum sont nettoyés, dissolus dans un solvant conventionnel et stabilisés.
